# EUROPEAN PATENT SPECIFICATION

(11) **EP 3 554 290 B1**
(45) Date of publication and mention of the grant of the patent: **30.12.2020**
(21) Application number: 17804914.4
(22) Date of filing: 29.11.2017
(51) Int. Cl.: A24F 40/10, A24F 40/30, A24F 40/42

(54) **AN AEROSOL-GENERATING SYSTEM HAVING A CARTRIDGE AND A BYPASS AIR INLET**
AEROSOLERZEUGUNGSSYSTEM MIT EINER KARTUSCHE UND EINEM BYPASS-LUFTEINLASS
SYSTÈME DE PRODUCTION D'AÉROSOL AYANT UNE CARTOUCHE ET UNE ENTRÉE D'AIR DE DÉRIVATION

(30) Priority: 19.12.2016 EP 16205103
(43) Date of publication of application: 23.10.2019
(73) Proprietor: Philip Morris Products S.A., 2000 Neuchâtel (CH)
(72) Inventor: REEVELL, Tony, London EC2A 4NE (GB)
(74) Representative: Dowling, Ian
(86) International application number: PCT/EP2017/080862
(87) International publication number: WO 2018/114261

(56) References cited:
- WO-A1-2014/110119
- WO-A1-2016/159013
- US-A1- 2014 166 029
- US-A1- 2016 345 631

## Description

The present invention relates to an aerosol-generating system having a cartridge and an aerosol-generating device, the aerosol-generating device having a cavity for receiving the cartridge and a bypass air inlet. The invention finds particular application as an electrically operated smoking system.

One type of aerosol-generating system is an electrically operated smoking system. Known handheld electrically operated smoking systems typically comprise an aerosol-generating device comprising a battery, control electronics and an electric heater for heating an aerosol-forming substrate. The aerosol-forming substrate may be contained within part of the aerosol-generating device. For example, the aerosol-generating device may comprise a liquid storage portion in which a liquid aerosol-forming substrate, such as a nicotine solution, is stored. Such devices, often referred to as 'e-cigarettes', typically contain sufficient liquid aerosol-forming substrate to provide a number of puffs equivalent to consuming multiple conventional cigarettes.

WO 2014/110119 A1 describes an electronic cigarette comprising a lithium battery, an atomizer, a refillable propylene glycol container, and a replaceable nicotine cartridge. The electronic cigarette also comprises an electronic control circuit and a sensor, which may be a pressure or flow sensor. The atomizer is coupled to the lithium battery through the electronic control circuit so that power is applied to the atomizer when a user draws on a mouthpiece of the electronic cigarette.

Document WO 2016/159013 discloses a non-combustion type flavor suction device comprising a flow path of aerosol generated by an atomization unit, and an additional air flow path to modify the overall resistance to draw.

In an attempt to provide e-cigarette users with an experience that more closely simulates the experience of consuming a conventional cigarette some devices have attempted to combine an e-cigarette configuration with a tobacco-based substrate to impart a tobacco taste to the aerosol inhaled by the user. The tobacco-based substrate may be provided in a removable cartridge. However, in such devices it may be possible for a user to use the device without the cartridge inserted, which may result in a diminished user experience.

It would be desirable to provide an aerosol-generating system that mitigates or eliminates at least some of these problems with known devices.

According to the present invention there is provided an aerosol-generating system according to claim 1. Said system comprises a cartridge, a liquid aerosol-forming substrate, and an aerosol-generating device. The cartridge comprises a cartridge housing and a solid aerosol-forming substrate positioned within the cartridge housing. The aerosol-generating device comprises a cavity for receiving at least a portion of the cartridge, an airflow inlet, and an airflow sensor positioned between the airflow inlet and the cavity, the airflow sensor in fluid communication with the airflow inlet and the cavity. The aerosol-generating device further comprises a bypass air inlet in fluid communication with the cavity, an electric heater configured to heat the liquid aerosol-forming substrate, a power supply, and a controller. The controller is configured to control a supply of electrical power from the power supply to the electric heater in response to a signal from the airflow sensor indicative of airflow across the airflow sensor. The aerosol-generating system is configured so that the cartridge housing substantially prevents airflow through the bypass air inlet when the cartridge is received within the cavity.

The liquid aerosol-forming substrate may form part of the cartridge or part of the aerosol-generating device.

As used herein, the term "aerosol-forming substrate" is used to describe a substrate capable of releasing volatile compounds, which can form an aerosol. The aerosols generated from aerosol-forming substrates of aerosol-generating systems according to the invention may be visible or invisible and may include vapours (for example, fine particles of substances, which are in a gaseous state, that are ordinarily liquid or solid at room temperature) as well as gases and liquid droplets of condensed vapours.

Advantageously, when a user draws on the aerosol-generating device without the cartridge inserted into the cavity, air is drawn into the aerosol-generating device via the bypass air inlet. In this case, there is insufficient airflow across the airflow sensor to trigger the airflow sensor. Therefore, advantageously, when a user draws on the aerosol-generating device without the cartridge inserted into the cavity, the controller does not activate the electric heater.

Advantageously, when the cartridge is received within the cavity the bypass air inlet is blocked by the cartridge housing. Therefore, when a user draws on the aerosol-generating device with the cartridge inserted into the cavity, air can only flow into the aerosol-generating device via the airflow inlet. In this case, there is sufficient airflow across the airflow sensor to trigger the airflow sensor. Therefore, advantageously, when a user draws on the aerosol-generating device with the cartridge inserted into the cavity, the controller activates the electric heater.

The aerosol-generating system may comprise an airflow outlet. When the cartridge is received within the cavity, the aerosol-generating system comprises an airflow path extending through the aerosol-generating system from the airflow inlet to the airflow outlet.

The bypass air inlet may be positioned on a sidewall of the cavity. A sidewall of the cavity is a wall along which the cartridge housing slides when the cartridge is inserted into the cavity. Advantageously, providing the bypass air inlet on a sidewall of the cavity may facilitate obstruction of the bypass air inlet by the cartridge housing when the cartridge is received within the cavity.

The bypass air inlet may comprise a plurality of bypass air inlets, wherein the aerosol-generating system is configured so that the cartridge housing substantially prevents airflow through each of the bypass air inlets when the cartridge is received within the cavity. Advantageously, providing a plurality of bypass air inlets may increase the ratio of airflow through the bypass air inlets to airflow through the airflow inlet when the cartridge is not received within the cavity. Therefore, advantageously, this arrangement may further reduce the airflow across the airflow sensor when a user draws on the aerosol-generating system without the cartridge received within the cavity.

The aerosol-generating device may comprise a liquid storage section, wherein the liquid aerosol-forming substrate is positioned within the liquid storage section. Preferably, the aerosol-generating device comprises a power supply section comprising the power supply and the controller, wherein the liquid storage section is configured for removable attachment to the power supply section.

The liquid storage section may comprise a porous carrier material, wherein the liquid aerosol-forming substrate is provided on the porous carrier material. Advantageously, providing the liquid aerosol-forming substrate on a porous carrier material may reduce the risk of the liquid aerosol-forming substrate leaking from the liquid storage section.

The aerosol-generating system may further comprise a liquid transfer element configured so that, in use, liquid aerosol-forming substrate is transported by capillary action along the liquid transfer element from the liquid storage section to the electric heater. In embodiments in which the liquid storage section comprises a porous carrier material, the liquid transfer element is configured to transport liquid aerosol-forming substrate from the porous carrier material to the electric heater.

The electric heater may be provided separately from one or both of the liquid storage section and the power supply section. Preferably, the liquid storage section, the electric heater and, where present, the liquid transfer element are provided together in a vaporiser section. Preferably, the vaporiser section comprises a vaporiser housing forming part of a device housing, wherein the vaporiser housing comprises an upstream end configured for connection to the power supply section and a downstream end defining a cavity configured to receive the cartridge assembly. Advantageously, providing the liquid storage section, the electric heater and, where present, the liquid transfer element in a single vaporiser section separate from the power supply section may facilitate replacement of the vaporiser section (for example, when the liquid aerosol-forming substrate has been depleted) without the need to replace the power supply section.

The cartridge may comprise the liquid aerosol-forming substrate positioned within the cartridge housing.

The cartridge may comprise a porous carrier material, wherein the liquid aerosol-forming substrate is provided on the porous carrier material. Advantageously, providing the liquid aerosol-forming substrate on a porous carrier material may reduce the risk of the liquid aerosol-forming substrate leaking from the cartridge.

The aerosol-generating device may comprise a liquid transfer element configured to transfer liquid aerosol-forming substrate from the cartridge to the electric heater when the cartridge is received within the cavity. In embodiments in which the cartridge comprises a porous carrier material, the liquid transfer element is configured to transport liquid aerosol-forming substrate from the porous carrier material to the electric heater.

The cartridge may comprise a seal. The seal may be a removable seal or a frangible seal. Advantageously, a seal may prevent the loss of volatile compounds from one or both of the solid aerosol-forming substrate and the liquid aerosol-forming substrate. The cartridge may comprise a seal extending across a first end of the cartridge housing. Preferably, the first end is the upstream end of the cartridge housing. The seal may be secured to the cartridge housing about a periphery of the seal. The seal may be secured to the cartridge housing by at least one of an adhesive and a weld, such as an ultrasonic weld. The seal is preferably formed from a sheet material. The sheet material may comprise at least one of a polymeric film and a metallic foil. In embodiments in which the seal is a removable seal, the removable seal may be configured for removal from the cartridge by a user before combining the cartridge with the aerosol-generating device. The removable seal may comprise a pull tab to facilitate removal of the seal.

In embodiments in which the seal is a frangible seal, preferably the aerosol-generating device comprises a piercing element configured to pierce the frangible seal when the aerosol-generating device receives the cartridge. Advantageously, a piercing element may automatically pierce the frangible seal when the aerosol-generating device and the heater section are combined with the cartridge.

At least a portion of the electric heater may form the piercing element. The electric heater may be in the form of a heater blade configured to pierce the frangible seal.

The piercing element may be formed separately from the electric heater. The piercing element may comprise a hollow shaft portion and a piercing portion at an end of the hollow shaft portion. Advantageously, a hollow shaft portion may allow airflow through the hollow shaft portion during use of the aerosol-generating system. The piercing portion may comprise an airflow aperture extending through the piercing portion and in fluid communication with the interior of the hollow shaft portion. At least a portion of the interior of the hollow shaft portion may define an airflow passage extending along at least a portion of the hollow shaft portion. The airflow passage is in fluid communication with the airflow inlet, the airflow sensor and the cavity. The airflow sensor may be positioned upstream of the airflow passage. The airflow sensor may be positioned downstream of the airflow passage.

At least a portion of the electric heater may be positioned inside the hollow shaft portion. At least a portion of the electric heater may extend transversely across a portion of the airflow passage. Preferably, the electric heater and the hollow shaft portion are configured so that, during use, airflow through the airflow passage passes across the portion of the electric heater positioned inside the hollow shaft portion.

In embodiments in which the aerosol-generating device comprises a liquid transfer element, a first portion of the liquid transfer element may be positioned inside the hollow shaft portion. The first portion of the liquid transfer element may extend transversely across a portion of the airflow passage. Preferably, the liquid transfer element and the hollow shaft portion are configured so that, during use, airflow through the airflow passage passes across the first portion of the liquid transfer element.

The electric heater may comprise a resistive heating coil. Preferably, the resistive heating coil is at least partially wound around the first portion of the liquid transfer element.

The liquid transfer element may extend through a first aperture in the hollow shaft portion, wherein a second portion of the liquid transfer element overlies an outer surface of the hollow shaft portion. The second portion of the liquid transfer element is preferably a first end of the liquid transfer element. The liquid transfer element may extend through a second aperture in the hollow shaft portion, wherein a third portion of the liquid transfer element overlies the outer surface of the hollow shaft portion. The second aperture is preferably opposite the first aperture. The third portion of the liquid transfer element is preferably a second end of the liquid transfer element. The first portion of the liquid transfer element is preferably an intermediate portion of the liquid transfer element between the second and third portions.

The aerosol-generating device may comprise a securing ring positioned around part of the hollow shaft portion, wherein at least part of the second portion of the liquid transfer element is positioned between the securing ring and the hollow shaft portion. In those embodiments in which the liquid transfer element comprises a third portion overlying the outer surface of the hollow shaft portion, preferably at least part of the third portion of the liquid transfer element is positioned between the securing ring and the hollow shaft portion.

In embodiments in which the cartridge comprises a porous carrier material, the porous carrier material may have an annular shape defining a passage through the porous carrier material. When the cartridge is received within the cavity, the passage defined through the porous carrier material may form part of an airflow path through the aerosol-generating system.

Preferably, the aerosol-generating system is configured so that the piercing element is at least partially received within the passage when the aerosol-generating device receives the cartridge. Preferably, the aerosol-generating system is configured so that the second portion of the liquid transfer element contacts an inner surface of the porous carrier material when the piercing portion is at least partially received within the passage. In embodiments in which the liquid transfer element comprises a third portion, preferably the aerosol-generating system is configured so that the third portion of the liquid transfer element contacts the inner surface of the porous carrier material when the piercing portion is at least partially received within the passage.

Preferably, the piercing portion is tapered and comprises a maximum diameter at a first end of the piercing portion adjacent the hollow shaft portion. Preferably, the piecing portion comprises a minimum diameter at a second end of the piercing portion. The second end of the piercing portion is configured to pierce the frangible seal of the cartridge.

Preferably, the hollow shaft portion has a first diameter adjacent the first end of the piercing portion, wherein the first diameter of the hollow shaft portion is less than the maximum diameter of the piercing portion. In embodiments in which the aerosol-generating device comprises a liquid transfer element having a second portion overlying an outer surface of the hollow shaft portion, preferably the maximum thickness of the second portion of the liquid transfer element is equal to or less than the difference between the maximum diameter and the first diameter. In embodiments in which the liquid transfer element has a third portion overlying the outer surface of the hollow shaft portion, preferably the maximum combined thickness of the second and third portions of the liquid transfer element is equal to or less than the difference between the maximum diameter and the first diameter. Advantageously, such arrangements may reduce stress on the liquid transfer element when the cartridge is combined with the aerosol-generating device, particularly in embodiments in which the piercing element is received within a passage extending through the porous carrier material.

In embodiments in which the cartridge comprises a porous carrier material, the cartridge may comprise an airflow channel positioned between the porous carrier material and the cartridge housing. Preferably, a downstream end of the airflow channel is in fluid communication with the solid aerosol-forming substrate. The airflow channel may be in addition to, or alternative to, a passage extending through the porous carrier material.

The cartridge housing is preferably tubular and comprises a first, upstream end and a second, downstream end. Preferably, the solid aerosol-forming substrate is positioned within the downstream end. Preferably, the liquid aerosol-forming substrate is positioned within the upstream end.

In embodiments in which the cartridge comprises a porous carrier material on which the liquid aerosol-forming substrate is positioned, the porous carrier material may be positioned directly within the cartridge housing. Preferably, the porous carrier material is retained within the cartridge housing by an interference fit.

The porous carrier material may be positioned within a liquid storage housing, wherein the liquid storage housing is positioned within the cartridge housing. Preferably, the liquid storage housing is retained within the cartridge housing by an interference fit.

In embodiments in which the cartridge comprises an airflow channel positioned between the porous carrier material and the cartridge housing, an outer surface of the liquid storage housing may be shaped to define the airflow channel between the cartridge housing and the liquid storage housing when the liquid storage housing is received within the cartridge housing. The outer surface of the liquid storage housing may comprise a groove to define the airflow channel when the liquid storage housing is received within the cartridge housing.

The liquid storage housing may be tubular. In embodiments in which the cartridge comprises a removable seal or a frangible seal, the seal may extend across the upstream end of the liquid storage housing. Preferably, the seal is secured to the liquid storage housing instead of the cartridge housing.

The tubular liquid storage housing may have an open upstream end and a closed downstream end. In embodiments in which the cartridge comprises a removable seal or a frangible seal, the seal may extend across the upstream end of the liquid storage housing so that the porous carrier material is positioned between the seal and the closed end.

The solid aerosol-forming substrate may be retained in the cartridge housing by an interference fit.

The cartridge may comprise a filter positioned downstream of the solid aerosol-forming substrate. The filter may comprise a plug of filter material positioned within the downstream end of the cartridge housing. The plug of filter material may be retained within the cartridge housing by an interference fit. The filter may comprise a sheet material extending across a downstream opening of the cartridge housing. The sheet material may comprise a mesh. The sheet material may be secured to the cartridge housing by at least one of an adhesive and a weld, such as an ultrasonic weld. The filter may retain the solid aerosol-forming substrate in the cartridge housing.

The aerosol-generating system may comprise a mouthpiece. In embodiments in which the aerosol-generating system comprises at least one airflow outlet, preferably the mouthpiece comprises the at least one airflow outlet. The mouthpiece may form part of the cartridge. The mouthpiece may form part of the aerosol-generating device. The mouthpiece may be formed separately from the cartridge and the aerosol-generating device, wherein at least one of the cartridge and the aerosol-generating device is configured to receive the mouthpiece.

The solid aerosol-forming substrate may comprise tobacco. The solid aerosol-forming substrate may comprise a tobacco-containing material containing volatile tobacco flavour compounds which are released from the substrate upon heating.

The solid aerosol-forming substrate may comprise tobacco containing deprotonated nicotine. Deprotonating the nicotine within tobacco may advantageously increase the volatility of the nicotine. Nicotine may be deprotonated by subjecting the tobacco to an alkalising treatment.

The solid aerosol-forming substrate may comprise a non-tobacco material. The solid aerosol-forming substrate may comprise tobacco-containing material and non-tobacco containing material.

The solid aerosol-forming substrate may include at least one aerosol-former. As used herein, the term 'aerosol former' is used to describe any suitable known compound or mixture of compounds that, in use, facilitates formation of an aerosol. Suitable aerosol-formers include, but are not limited to: polyhydric alcohols, such as propylene glycol, triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate

Preferred aerosol formers are polyhydric alcohols or mixtures thereof, such as propylene glycol, triethylene glycol, 1,3-butanediol and, most preferred, glycerine.

The solid aerosol-forming substrate may comprise a single aerosol former. Alternatively, the solid aerosol-forming substrate may comprise a combination of two or more aerosol formers.

The solid aerosol-forming substrate may have an aerosol former content of greater than 5 percent on a dry weight basis.

The solid aerosol-forming substrate may have an aerosol former content of between approximately 5 percent and approximately 30 percent on a dry weight basis.

The solid aerosol-forming substrate may have an aerosol former content of approximately 20 percent on a dry weight basis.

The liquid aerosol-forming substrate may comprise a tobacco-containing material comprising volatile tobacco flavour compounds which are released from the liquid upon heating. The liquid aerosol-forming substrate may comprise a non-tobacco material. The liquid aerosol-forming substrate may include water, solvents, ethanol, plant extracts and natural or artificial flavours. Preferably, the liquid aerosol-forming substrate comprises an aerosol former. Suitable aerosol formers include polyhydric alcohols or mixtures thereof, such as propylene glycol, triethylene glycol, 1,3-butanediol and glycerine.

The liquid aerosol-forming substrate may comprise nicotine.

The liquid aerosol-forming substrate may be free from nicotine. In such embodiments, the vaporised liquid aerosol-forming substrate may be drawn through the solid aerosol-forming substrate during use to strip one or more volatile compounds from the solid aerosol-forming substrate. The vaporised liquid aerosol-forming substrate may strip nicotine from the solid-aerosol-forming substrate. A solid aerosol-forming substrate comprising tobacco containing deprotonated nicotine may be particularly suited to embodiments in which the liquid aerosol-forming substrate is free from nicotine.

The electric heater may comprise a resistive heating coil. The aerosol-generating device may comprise a heater support, wherein the resistive heating coil is at least partially wound around the heater support.

The electric heater may comprise a resistive heating mesh.

The resistive heating mesh may comprise a plurality of electrically conductive filaments. The electrically conductive filaments may be substantially flat. As used herein, "substantially flat" means formed in a single plane and not wrapped around or otherwise conformed to fit a curved or other non-planar shape. A flat heating mesh can be easily handled during manufacture and provides for a robust construction.

The electrically conductive filaments may define interstices between the filaments and the interstices may have a width of between about 10 micrometres and about 100 micrometres. Preferably the filaments give rise to capillary action in the interstices, so that in use, liquid aerosol-forming substrate is drawn into the interstices, increasing the contact area between the heater assembly and the liquid.

The electrically conductive filaments may form a mesh of size between about 160 Mesh US and about 600 Mesh US (+/- 10%) (that is, between about 160 and about 600 filaments per inch (+/- 10%)). The width of the interstices is preferably between about 75 micrometres and about 25 micrometres. The percentage of open area of the mesh, which is the ratio of the area of the interstices to the total area of the mesh is preferably between about 25 percent and about 56 percent. The mesh may be formed using different types of weave or lattice structures. The electrically conductive filaments may be an array of filaments arranged parallel to one another.

The electrically conductive filaments may have a diameter of between about 8 micrometres and about 100 micrometres, preferably between about 8 micrometres and about 50 micrometres, and more preferably between about 8 micrometres and about 39 micrometres.

The resistive heating mesh may cover an area of less than or equal to about 25 square millimetres. The resistive heating mesh may be rectangular. The resistive heating mesh may be square. The resistive heating mesh may have dimensions of about 5 millimetres by about 2 millimetres.

The electrically conductive filaments may comprise any suitable electrically conductive material. Suitable materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, constantan, nickel-, cobalt-, chromium-, aluminium-titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. Timetal® is a registered trade mark of Titanium Metals Corporation. The filaments may be coated with one or more insulators. Preferred materials for the electrically conductive filaments are 304, 316, 304L, and 316L stainless steel, and graphite.

The electrical resistance of the resistive heating mesh is preferably between about 0.3 and about 4 Ohms. More preferably, the electrical resistance of the mesh is between about 0.5 and about 3 Ohms, and more preferably about 1 Ohm.

In embodiments in which the electric heater comprises a resistive heating coil, the pitch of the coil is preferably between about 0.5 millimetres and about 1.5 millimetres, and most preferably about 1.5 millimetres. The pitch of the coil means the spacing between adjacent turns of the coil. The coil may comprise fewer than six turns, and preferably has fewer than five turns. The coil may be formed from an electrically resistive wire having a diameter of between about 0.10 millimetres and about 0.15 millimetres, preferably about 0.125 millimetres. The electrically resistive wire is preferably formed of 904 or 301 stainless steel. Examples of other suitable metals include titanium, zirconium, tantalum and metals from the platinum group. Examples of other suitable metal alloys include, Constantan, nickel-, cobalt-, chromium-, aluminium- titanium-zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. The resistive heating coil may also comprise a metal foil, such as an aluminium foil, which is provided in the form of a ribbon.

In embodiments in which the aerosol-generating system comprises a porous carrier material, the porous carrier material may comprise any suitable material or combination of materials which is permeable to the liquid aerosol-forming substrate and allows the liquid aerosol-forming substrate to migrate through the porous carrier material. Preferably, the material or combination of materials is inert with respect to the liquid aerosol-forming substrate. The porous carrier material may or may not be a capillary material. The porous carrier material may comprise a hydrophilic material to improve distribution and spread of the liquid aerosol-forming substrate. This may assist with consistent aerosol formation. The particular preferred material or materials will depend on the physical properties of the liquid aerosol-forming substrate. Examples of suitable materials are a capillary material, for example a sponge or foam material, ceramic- or graphite-based materials in the form of fibres or sintered powders, a foamed metal or plastics material, a fibrous material, for example made of spun or extruded fibres, such as cellulose acetate, polyester, or bonded polyolefin, polyethylene, terylene or polypropylene fibres, nylon fibres or ceramic. The porous carrier material may have any suitable porosity so as to be used with different liquid physical properties.

In embodiments in which the aerosol-generating system comprises a liquid transfer element, the liquid transfer element may comprise any suitable material or combination of materials which is able to convey the liquid aerosol-forming substrate along its length. The liquid transfer element may be formed from a porous material, but this need not be the case. The liquid transfer element may be formed from a material having a fibrous or spongy structure. The liquid transfer element preferably comprises a bundle of capillaries. For example, the liquid transfer element may comprise a plurality of fibres or threads or other fine bore tubes. The liquid transfer element may comprise sponge-like or foam-like material. Preferably, the structure of the liquid transfer element forms a plurality of small bores or tubes, through which the liquid aerosol-forming substrate can be transported by capillary action. The particular preferred material or materials will depend on the physical properties of the liquid aerosol-forming substrate. Examples of suitable capillary materials include a sponge or foam material, ceramic- or graphite-based materials in the form of fibres or sintered powders, foamed metal or plastics material, a fibrous material, for example made of spun or extruded fibres, such as cellulose acetate, polyester, or bonded polyolefin, polyethylene, terylene or polypropylene fibres, nylon fibres, ceramic, glass fibres, silica glass fibres, carbon fibres, metallic fibres of medical grade stainless steel alloys such as austenitic 316 stainless steel and martensitic 440 and 420 stainless steels. The liquid transfer element may have any suitable capillarity so as to be used with different liquid physical properties. The liquid aerosol-forming substrate has physical properties, including but not limited to viscosity, surface tension, density, thermal conductivity, boiling point and vapour pressure, which allow the liquid aerosol-forming substrate to be transported through the liquid transfer element. The liquid transfer element may be formed from heat-resistant material. The liquid transfer element may comprise a plurality of fibre strands. The plurality of fibre strands may be generally aligned along a length of the liquid transfer element.

In embodiments in which the aerosol-generating system comprises a porous carrier material and a liquid transfer element, the porous carrier material and the liquid transfer element may comprise the same material. Preferably, the porous carrier material and the liquid transfer element comprise different materials.

Preferably, the cartridge is configured to be retained within the cavity by an interference fit. Each of the cartridge and the cavity may have any suitable cross-sectional shape. Preferably, a cross-sectional shape of the cartridge is substantially the same as a cross-sectional shape of the cavity. Suitable cross-sectional shaped include circular, semi-circular, polygonal, such as rectangular, including square, and irregular shapes.

The cartridge housing may be formed from any suitable material or combination of materials. Suitable materials include, but are not limited to, aluminium, polyether ether ketone (PEEK), polyimides, such as Kapton®, polyethylene terephthalate (PET), polyethylene (PE), high-density polyethylene (HDPE), polypropylene (PP), polystyrene (PS), fluorinated ethylene propylene (FEP), polytetrafluoroethylene (PTFE), polyoxymethylene (POM), epoxy resins, polyurethane resins, vinyl resins, liquid crystal polymers (LCP) and modified LCPs, such as LCPs with graphite or glass fibres.

The power supply may comprise a battery. For example, the power supply may be a nickel-metal hydride battery, a nickel cadmium battery, or a lithium based battery, for example a lithium-cobalt, a lithium-iron-phosphate or a lithium-polymer battery. The power supply may alternatively be another form of charge storage device such as a capacitor. The power supply may require recharging and may have a capacity that allows for the storage of enough energy for use of the aerosol-generating device with more than one cartridge.

The invention is further described, by way of example only, with reference to the accompanying drawings in which:
Figure 1 shows a perspective view of an aerosol-generating system according to a first embodiment of the present invention;
Figure 2 shows a cross-sectional view of the aerosol-generating system of Figure 1 with the cartridge received in the cavity;
Figure 3 shows the aerosol-generating device of Figure 2 without the cartridge inserted into the cavity;
Figure 4 shows a cross-sectional view of an aerosol-generating device according to a second embodiment of the present invention;
Figure 5 shows a cross-sectional view of an aerosol-generating system according to a third embodiment of the present invention, with the cartridge separate from the aerosol-generating device;
Figure 6 shows a cross-sectional view of the aerosol-generating system of Figure 5 with the cartridge received within the cavity; and
Figure 7 shows an alternative arrangement of the heater section of the aerosol-generating system of Figure 5.

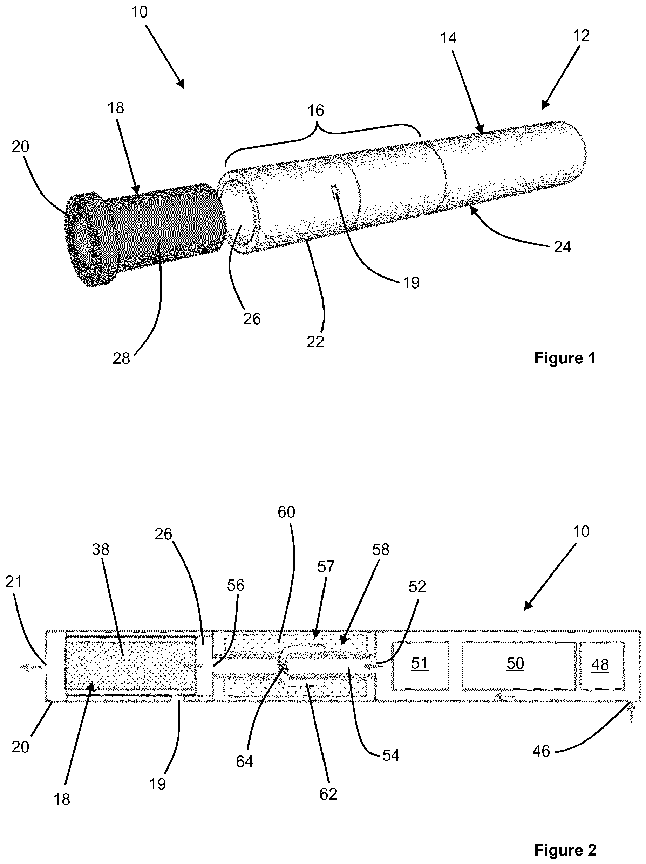

Figure 1 shows a perspective view of an aerosol-generating system 10 according to a first embodiment of the present invention. The aerosol-generating system 10 comprises an aerosol-generating device 12 comprising a power supply section 14 and a vaporiser section 16. The aerosol-generating system 10 further comprises a cartridge 18 and a mouthpiece 20 forming part of the cartridge 18. The vaporiser section 16 comprises a vaporiser housing 22 that forms part of a device housing 24. A downstream end of the vaporiser housing 22 defines a cavity 26 for receiving the cartridge 18. The vaporiser housing 22 also defines a bypass air inlet 19 in fluid communication with the cavity 26.

Figure 2 shows a cross-sectional view of the aerosol-generating system 10 with the cartridge 18 received within the cavity 26. The cartridge 18 comprises a cartridge housing 28 and a solid aerosol-forming substrate 38 positioned within the cartridge housing 28. A downstream end of the solid aerosol-forming substrate 38 is in fluid communication with an airflow outlet 21 defined by the mouthpiece 20.

The power supply section 14 comprises a system air inlet 46 for admitting air into the power supply section 14. Positioned within the power supply section 14 are a controller 48, a power supply 50 and an airflow sensor 51. The airflow sensor 51 is configured to provide a signal to the controller 48 when airflow across the airflow sensor 51 exceeds a predetermined threshold.

The vaporiser section 16 comprises a vaporiser air inlet 52 for receiving air from the power supply section 14, an airflow passage 54 in fluid communication with the vaporiser air inlet 52 at its upstream end, and a cavity air inlet 56 providing fluid communication between the downstream end of the airflow passage 54 and the cavity 26.

The vaporiser section 16 further comprises a liquid storage section 57 comprising a liquid aerosol-forming substrate 58 sorbed into an annular porous carrier material 60 positioned outside of the airflow passage 54. A liquid transfer element 62 comprising a capillary wick has first and second ends positioned in contact with the porous carrier material 60 and a central portion positioned within the airflow passage 54. Liquid aerosol-forming substrate 58 is wicked by capillary action along the capillary wick from the porous carrier material 60 to the central portion of the capillary wick.

The vaporiser section 16 also comprises an electric heater 64 comprising a resistive heating coil wound around the central portion of the capillary wick. During operation of the aerosol-generating system 10, the controller 48 controls a supply of electrical energy from the power supply 50 to the electric heater 64 to heat and vaporise liquid aerosol-forming substrate 58 from the central portion of the capillary wick.

When the cartridge 18 is received within the cavity 26, as shown in Figure 2, the cartridge housing 28 obstructs the bypass air inlet 19 to prevent airflow into the cavity 26 via the bypass air inlet 19. In this configuration, when a user draws on the mouthpiece 20, air is drawn into the system through the system air inlet 46. As airflow enters the power supply section 14 through the airflow inlet 46, airflow across the airflow sensor 51 activates the airflow sensor 51. The activated airflow sensor 51 provides a signal to the controller 48 to indicate airflow through the aerosol-generating system 10 via the airflow inlet 46. Upon receipt of the signal from the airflow sensor 51, the controller 48 controls a supply of electrical energy from the power supply 50 to the electric heater 64 to vaporise liquid aerosol-forming substrate 58 from the liquid transfer element 62. Airflow from the power supply section 14 flows through the vaporiser air inlet 52 and into the airflow passage 54 where vaporised liquid aerosol-forming substrate 58 is entrained in the airflow. The airflow then flows through the cavity air inlet 56, into the cavity 26 and the cartridge 18 where volatile compounds from the cartridge aerosol-forming substrate 38 are entrained in the airflow. The airflow then flows out of the cartridge 18 and out of the aerosol-generating system 10 through the air outlet 21 to deliver to the user the vaporised liquid aerosol-forming substrate 58 and the volatile compounds from the cartridge aerosol-forming substrate 38.

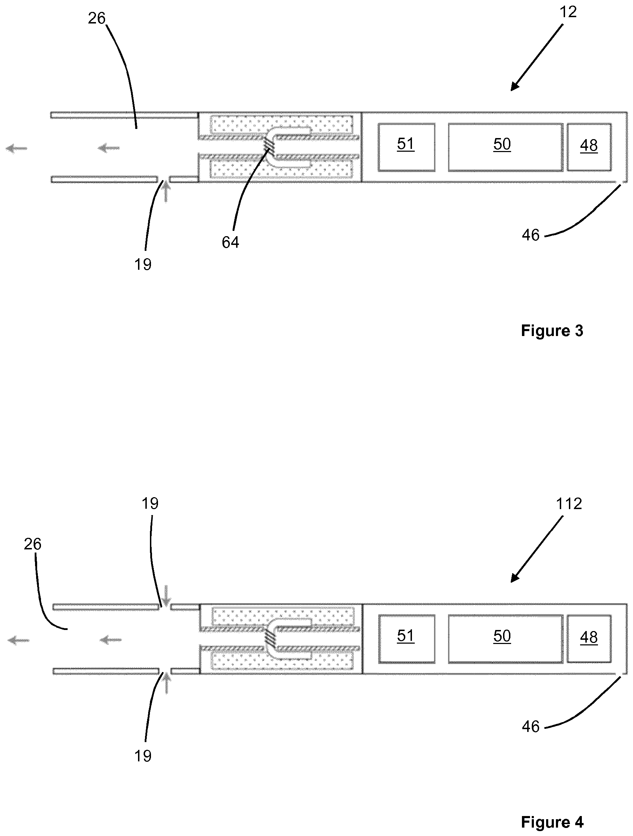

When the cartridge 18 is not received within the cavity 26, as shown in Figure 3, the bypass air inlet 19 is unobstructed. Therefore, in this configuration, when a user attempts to draw on a downstream end of the aerosol-generating device, airflow enters the cavity 26 via the bypass air inlet 19. As such, insufficient airflow is drawn into the power supply section 14 via the airflow inlet 46 to activate the airflow sensor 51. Therefore, when the cartridge 18 is not received in the cavity 26, a user cannot activate the electric heater 64 by drawing on the downstream end of the aerosol-generating device 12.

Figure 4 shows an aerosol-generating device 112 according to a second embodiment of the present invention. The aerosol-generating device 112 is substantially the same as the aerosol-generating device 12 shown in Figures 1 to 3 and like reference numerals are used to designate like parts. The aerosol-generating device 112 differs by the addition of a second bypass air inlet 19. Providing an additional bypass air inlet 19 may increase the ratio of airflow through the bypass air inlets 19 to airflow through the airflow inlet 46 when the cartridge 18 is not received within the cavity 26. Therefore, advantageously, this arrangement may further reduce the airflow across the airflow sensor 51 when a user draws on the aerosol-generating device 112 without the cartridge 18 received within the cavity 26.

Figure 4 shows an aerosol-generating system 200 according to a third embodiment of the present invention. The aerosol-generating system 200 is substantially the same as the aerosol-generating system 10 shown in Figures 1 to 3 and like reference numerals are used to designate like parts.

The aerosol-generating system 200 differs by the location of the liquid aerosol-forming substrate 58, which is positioned within the cartridge 218.

The cartridge 218 comprises a cartridge housing 28, and a solid aerosol-forming substrate 38 and a liquid aerosol-forming substrate 58 both positioned within the cartridge housing 36. The solid aerosol-forming substrate 38 comprises a tobacco plug positioned within the downstream end of the cartridge housing 28.

The liquid aerosol-forming substrate 58 is provided on a porous carrier material 260 positioned within the upstream end of the cartridge housing 28. A first frangible seal 248 extends across the upstream end of the porous carrier material 260 and a second frangible seal 250 extends across the downstream end of the porous carrier material 260. The porous carrier material 260 has an annular shape and defines a passage 254 through the porous carrier material 260, the passage 254 extending between the first and second frangible seals 248, 250.

The aerosol-generating device 212 comprises a power supply section housing a controller 48, a power supply 50 and an airflow sensor 51, as described with respect to the aerosol-generating system 10 of Figures 1 to 3. The device housing 214 defines a cavity 26 for receiving the cartridge 218.

The aerosol-generating device 212 further comprises a heater section 263 positioned at an upstream end of the cavity 26. The heater section 263 comprises an electric heater 64 in the form of a resistive heating coil. During use, the controller 48 controls a supply of electrical power from the power supply 50 to the electric heater 64. The heater section 26 further comprises a liquid transfer element 62 in the form of a capillary wick, the resistive heating coil wound around a first portion of the liquid transfer element 62.

The electric heater 64 and the liquid transfer element 62 are supported by a piercing element extending from an upstream end wall of the cavity 26. The piercing element comprises a hollow shaft portion 232 and a piercing portion 234. The electric heater 64 and the first portion of the liquid transfer element 62 are positioned in an airflow passage formed within the hollow shaft portion 232. Second and third portions of the liquid transfer element 62 extend through apertures in the hollow shaft portion 232, the second and third portions folded through a 90 degree angle so that they overlie an outer surface of the hollow shaft portion 232.

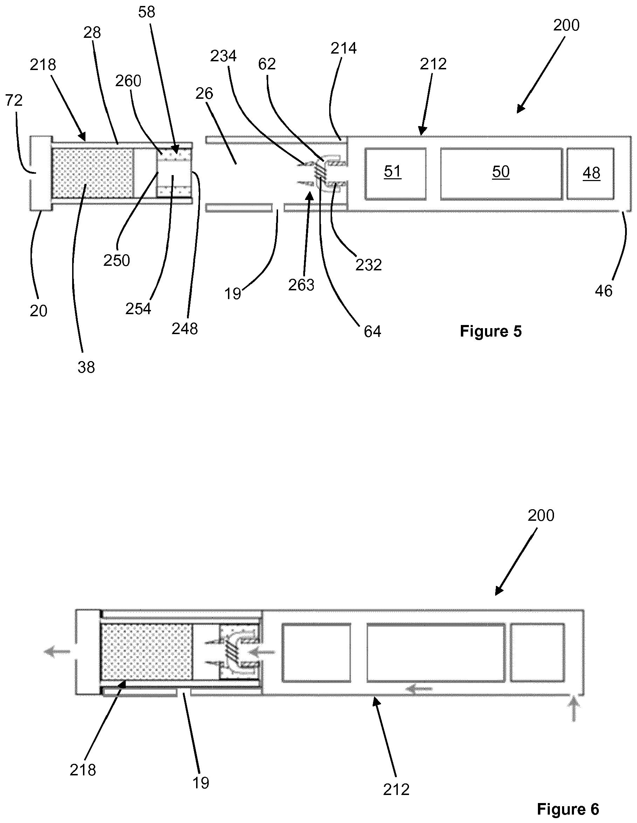

Figure 6 shows a cross-sectional view of the aerosol-generating system 200 after the cartridge 218 has been inserted into the cavity 26 of the aerosol-generating device 212. When the cartridge 218 is inserted into the cavity 26 the piercing portion 234 of the piercing element pierces the first and second frangible seals 248, 250. The heater section 263 is received within the passage 254 defined through the porous carrier material 260 so that the second and third portions of the liquid transfer element 62 contact the inner surface of the porous carrier material 260. Liquid aerosol-forming substrate 58 is wicked along the liquid transfer element 62 to the electric heater 64.

The operation of the aerosol-generating system 200 is identical to the operation of the aerosol-generating system 10 described with reference to Figures 1 to 3. That is, when the cartridge 218 is not received within the cavity 26, air flows into the cavity 26 via the bypass air inlet 19 when a user draws on the downstream end of the aerosol-generating device 212. When a user draws on the downstream end of the aerosol-generating system 200 with the cartridge 218 received within the cavity 26, airflow enters the aerosol-generating system 200 via the airflow inlet 46 and flows across the airflow sensor 51 to activate the aerosol-generating system 200.

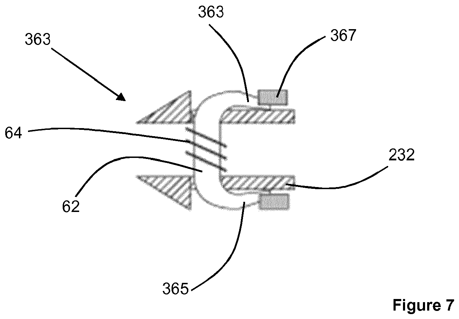

Figure 7 shows an alternative arrangement of a heater section 363 for the aerosol-generating system 200 of Figures 5 and 6. The heater section 363 is similar in construction to the heater section 263 described with reference to Figures 5 and 6 and like reference numerals are used to designate like parts.

The heater section 363 differs from the heater section 263 by the addition of a securing ring 367 positioned about the upstream end of the hollow shaft portion 232. The ends of the second and third portions 363, 365 of the liquid transfer element 62 are secured between the securing ring 367 and the hollow shaft portion 232 to retain the liquid transfer element 62 in the correct position during insertion and removal of the heater section 363 into and from the passage 254 defined through the porous carrier material 260.

## Claims

1. An aerosol-generating system (10) comprising:
a cartridge (18) comprising a cartridge housing (28) and a solid aerosol-forming substrate (38) positioned within the cartridge housing (28);
a liquid aerosol-forming substrate (58); and
an aerosol-generating device (12) comprising:
a cavity (26) for receiving at least a portion of the cartridge (18);
an airflow inlet (46);
an airflow sensor (51) positioned between the airflow inlet (46) and the cavity (26), the airflow sensor (51) in fluid communication with the airflow inlet (46) and the cavity (26);
a bypass air inlet (19) in fluid communication with the cavity (26);
an electric heater (64) configured to heat the liquid aerosol-forming substrate (58);
a power supply (50); and
a controller (48) configured to control a supply of electrical power from the power supply (50) to the electric heater (64) in response to a signal from the airflow sensor (51) indicative of airflow across the airflow sensor (51);
**characterized in that** the aerosol-generating system (10) is configured so that the cartridge housing (28) prevents airflow through the bypass air inlet (19) when the cartridge (18) is received within the cavity (26).

2. An aerosol-generating system (10) according to claim 1, wherein the bypass air inlet (19) is positioned on a sidewall of the cavity (26).

3. An aerosol-generating system (10) according to claim 1 or 2, wherein the bypass air inlet (19) comprises a plurality of bypass air inlets, and wherein the aerosol-generating system (10) is configured so that the cartridge housing (28) prevents airflow through each of the bypass air inlets when the cartridge (18) is received within the cavity (26).

4. An aerosol-generating system (10) according to claim 1, 2 or 3, wherein the aerosol-generating device (12) comprises a liquid storage section (57) and wherein the liquid aerosol-forming substrate (58) is positioned within the liquid storage section (57).

5. An aerosol-generating system (10) according to claim 4, wherein the liquid storage section (57) comprises a porous carrier material (60), and wherein the liquid aerosol-forming substrate (58) is provided on the porous carrier material (60).

6. An aerosol-generating system (10) according to any preceding claim, wherein the aerosol-generating device (12) further comprises a liquid transfer element (62) configured to transfer liquid aerosol-forming substrate (58) to the electric heater (64) during use of the aerosol-generating system (10).

7. An aerosol-generating system (10) according to claim 1, 2 or 3, wherein the cartridge (18) comprises the liquid aerosol-forming substrate (58) positioned within the cartridge housing (28).

8. An aerosol-generating system (200) according to claim 6, wherein the cartridge (218) comprises a porous carrier material (260), and wherein the liquid aerosol-forming substrate (58) is provided on the porous carrier material (260).

9. An aerosol-generating system (200) according to claim 8, wherein the aerosol-generating device (212) further comprises a liquid transfer element (62) configured to transfer liquid aerosol-forming substrate (58) from the porous carrier material (260) to the electric heater (64) during use of the aerosol-generating system (200).

10. An aerosol-generating system (200) according to claim 9, wherein the cartridge (218) further comprises a frangible seal (248, 250), and wherein the aerosol-generating device (212) further comprises a piercing element configured to pierce the frangible seal (248, 250) when the aerosol-generating device (212) receives the cartridge (218).

11. An aerosol-generating system (200) according to claim 10, wherein the piercing element comprises a hollow shaft portion (232) and a piercing portion (234) at an end of the hollow shaft portion (232), and wherein at least a portion of the electric heater (64) is positioned inside the hollow shaft portion (232).

12. An aerosol-generating system (200) according to claim 11, wherein a first portion of the liquid transfer element (62) is positioned inside the hollow shaft portion (232), and wherein the electric heater (64) comprises a resistive heating coil at least partially wound around the first portion of the liquid transfer element (62).

13. An aerosol-generating system (200) according to claim 12, wherein the liquid transfer element (62) extends through an aperture in the hollow shaft portion (232), and wherein a second portion of the liquid transfer element (62) overlies an outer surface of the hollow shaft portion (232).

14. An aerosol-generating system (200) according to claim 13, wherein the porous carrier material (260) has an annular shape defining a passage (254) through the porous carrier material (260), wherein the aerosol-generating system (200) is configured so that the piercing element is at least partially received within the passage (254) when the aerosol-generating device (212) receives the cartridge (218), and wherein the aerosol-generating system (200) is configured so that the second portion of the liquid transfer element (62) contacts an inner surface of the porous carrier material (260) when the piercing portion (234) is at least partially received within the passage (254).

15. An aerosol-generating system (200) according to claim 14, wherein the cartridge (218) comprises an airflow channel positioned between the porous carrier material (260) and the cartridge housing (28), and wherein a downstream end of the airflow channel is in fluid communication with the solid aerosol-forming substrate (38).

## Patentansprüche

1. Aerosolerzeugungssystem (10), aufweisend:
eine Patrone (18), die ein Patronengehäuse (28) und ein festes aerosolbildendes Substrat (38), das innerhalb des Patronengehäuses (28) positioniert ist, aufweist;
ein flüssiges aerosolbildendes Substrat (58); und
eine Aerosolerzeugungsvorrichtung (12), aufweisend:
einen Hohlraum (26), zum Aufnehmen von zumindest einem Abschnitt der Patrone (18);
einen Luftstromeinlass (46);
einen Luftstromsensor (51), der zwischen dem Luftstromeinlass (46) und dem Hohlraum (26) positioniert ist, wobei der Luftstromsensor (51) in Fluidverbindung mit dem Luftstromeinlass (46) und dem Hohlraum (26) steht;
ein Bypass-Lufteinlass (19) in Fluidverbindung mit dem Hohlraum (26);
eine elektrische Heizvorrichtung (64), die so ausgelegt ist, dass sie ein flüssiges aerosolbildendes Substrat (58) erwärmt;
eine Energieversorgung (50); und
einen Regler (48), der so ausgelegt ist, dass er eine Versorgung mit elektrischer Energie von der Energieversorgung (50) an die elektrische Heizvorrichtung (64) in Reaktion auf ein Signal von dem Luftstromsensor (51), der einen Luftstrom über den Luftstromsensor (51) anzeigt, regelt;
**dadurch gekennzeichnet, dass** das Aerosolerzeugungssystem (10) so ausgelegt ist, dass das Patronengehäuse (28) den Luftstrom durch den Bypass-Lufteinlass (19) verhindert, wenn die Patrone (18) innerhalb des Hohlraums (26) aufgenommen ist.

2. Aerosolerzeugungssystem (10) nach Anspruch 1, wobei der Bypass-Lufteinlass (19) an einer Seitenwand des Hohlraums (26) positioniert ist.

3. Aerosolerzeugungssystem (10) nach Anspruch 1 oder 2, wobei der Bypass-Lufteinlass (19) eine Vielzahl von Bypass-Lufteinlässen aufweist, und wobei das Aerosolerzeugungssystem (10) so ausgelegt ist, dass das Patronengehäuse (28) einen Luftstrom durch jeden der Bypass-Lufteinlässe verhindert, wenn die Patrone (18) in dem Hohlraum (26) aufgenommen ist.

4. Aerosolerzeugungssystem (10) nach Anspruch 1, 2 oder 3, wobei die Aerosolerzeugungsvorrichtung (12) einen Flüssigspeicherabschnitt (57) aufweist und wobei das flüssige aerosolbildende Substrat (58) innerhalb des Flüssigspeicherabschnitts (57) positioniert ist.

5. Aerosolerzeugungssystem (10) nach Anspruch 4, wobei der Flüssigspeicherabschnitt (57) ein poröses Trägermaterial (60) aufweist und wobei das flüssige aerosolbildende Substrat (58) auf dem porösen Trägermaterial (60) vorgesehen ist.

6. Aerosolerzeugungssystem (10) nach einem der vorhergehenden Ansprüche, wobei die Aerosolerzeugungsvorrichtung (12) ferner ein Flüssigkeitsübergabeelement (62) aufweist, das so ausgelegt ist, dass es während der Verwendung des Aerosolerzeugungssystems (10) das flüssige aerosolbildende Substrat (58) auf die elektrische Heizvorrichtung (64) überträgt.

7. Aerosolerzeugungssystem (10) nach Anspruch 1, 2 oder 3, wobei die Patrone(18) das flüssige aerosolbildende Substrat (58) aufweist, das innerhalb des Patronengehäuses (28) positioniert ist.

8. Aerosolerzeugungssystem (200) nach Anspruch 6, wobei die Patrone (218) ein poröses Trägermaterial (260) aufweist und wobei das flüssige aerosolbildende Substrat (58) auf dem porösen Trägermaterial (260) vorgesehen ist.

9. Aerosolerzeugungssystem (200) nach Anspruch 8, wobei die Aerosolerzeugungsvorrichtung (212) ferner ein Flüssigkeitsübergabeelement (62) aufweist, das so ausgelegt ist, dass es während der Verwendung des Aerosolerzeugungssystems (200) das flüssige aerosolbildende Substrat (58) von dem porösen Trägermaterial (260) zu der elektrischen Heizvorrichtung (64) überträgt.

10. Aerosolerzeugungssystem (200) nach Anspruch 9, wobei die Patrone (218) ferner eine fragmentierbare Dichtung (248, 250) aufweist und wobei die Aerosolerzeugungsvorrichtung (212) ferner ein Durchbohrungselement aufweist, das so ausgelegt ist, dass es die fragmentierbare Dichtung (248, 250) durchbohrt, wenn die Aerosolerzeugungsvorrichtung (212) die Patrone (218) aufnimmt.

11. Aerosolerzeugungssystem (200) nach Anspruch 10, wobei das Durchbohrungselement einen hohlen Schaftabschnitt (232) und einen Durchbohrungsabschnitt (234) an einem Ende des hohlen Schaftabschnitts (232) aufweist, und wobei zumindest ein Abschnitt der elektrischen Heizvorrichtung (64) innerhalb des hohlen Schaftabschnitts (232) positioniert ist.

12. Aerosolerzeugungssystem (200) nach Anspruch 11, wobei ein erster Abschnitt des Flüssigkeitsübergabeelements (62) innerhalb des hohlen Schaftabschnitts (232) positioniert ist und wobei die elektrische Heizvorrichtung (64) eine Widerstandsheizspule aufweist, die zumindest teilweise um den ersten Abschnitt des Flüssigkeitsübergabeelements (62) gewickelt ist.

13. Aerosolerzeugungssystem (200) nach Anspruch 12, wobei das Flüssigkeitsübergabeelement (62) durch eine Aussparung in dem hohlen Schaftabschnitt (232) verläuft und wobei ein zweiter Abschnitt des Flüssigkeitsübergabeelements (62) über einer Außenfläche des hohlen Schaftabschnitts (232) liegt.

14. Aerosolerzeugungssystem (200) nach Anspruch 13, wobei das poröse Trägermaterial (260) eine ringförmige Form hat, die einen Durchgang (254) durch das poröse Trägermaterial (260) definiert, wobei das Aerosolerzeugungssystem (200) so ausgelegt ist, dass das Durchbohrungselement zumindest teilweise in dem Durchgang (254) aufgenommen ist, wenn die Aerosolerzeugungsvorrichtung (212) die Patrone (218) aufnimmt, und wobei das Aerosolerzeugungssystem (200) so ausgelegt ist, dass der zweite Abschnitt des Flüssigkeitsübergabeelements (62) mit einer Innenfläche des porösen Trägermaterials (260) in Kontakt kommt, wenn der Durchbohrungsabschnitt (234) zumindest teilweise in dem Durchgang (254) aufgenommen ist.

15. Aerosolerzeugungssystem (200) nach Anspruch 14, wobei die Patrone (218) einen Luftstromkanal aufweist, der zwischen dem porösen Trägermaterial (260) und dem Patronengehäuse (28) positioniert ist, und wobei ein nachgeschaltetes Ende des Luftstromkanals (45) in Fluidverbindung mit dem festen aerosolbildenden Substrat (38) steht.

## Revendications

1. Système de génération d'aérosol (10) comprenant :
une cartouche (18) comprenant un logement de cartouche (28) et un substrat solide formant aérosol (38) positionné à l'intérieur du logement de cartouche (28) ;
un substrat liquide formant aérosol (58) ; et
un dispositif générateur d'aérosol (12) comprenant :
une cavité (26) pour recevoir au moins une partie de la cartouche (18) ;
une entrée d'écoulement d'air (46) ;
un capteur d'écoulement d'air (51) positionné entre l'entrée d'écoulement d'air (46) et la cavité (26), le capteur d'écoulement d'air (51) en communication fluidique avec l'entrée d'écoulement d'air (46) et la cavité (26) ;
une entrée d'air de dérivation (19) en communication fluidique avec la cavité (26) ;
un dispositif de chauffage électrique (64) configuré pour chauffer le substrat liquide formant aérosol (58) ;
une alimentation électrique (50) ; et
un contrôleur (48) configuré pour contrôler une alimentation en énergie électrique de l'alimentation électrique (50) au dispositif de chauffage électrique (64) en réponse à un signal du capteur d'écoulement d'air (51) indiquant un écoulement d'air à travers le capteur d'écoulement d'air (51) ;
**caractérisé en ce que** le système de génération d'aérosol (10) est configuré de telle sorte que le logement de cartouche (28) empêche l'écoulement d'air à travers l'entrée d'air de dérivation (19) lorsque la cartouche (18) est reçue dans la cavité (26).

2. Système de génération d'aérosol (10) selon la revendication 1, dans lequel l'entrée d'air de dérivation (19) est positionnée sur une paroi latérale de la cavité (26).

3. Système de génération d'aérosol (10) selon la revendication 1 ou 2, dans lequel l'entrée d'air de dérivation (19) comprend une pluralité d'entrées d'air de dérivation, et dans lequel le système de génération d'aérosol (10) est configuré de telle sorte que le logement de cartouche (28) empêche l'écoulement d'air à travers chacune des entrées d'air de dérivation lorsque la cartouche (18) est reçue dans la cavité (26).

4. Système de génération d'aérosol (10) selon la revendication 1, 2 ou 3, dans lequel le dispositif générateur d'aérosol (12) comprend une section de stockage de liquide (57) et dans lequel le substrat liquide formant aérosol (58) est positionné à l'intérieur de la section de stockage de liquide (57).

5. Système de génération d'aérosol (10) selon la revendication 4, dans lequel la section de stockage de liquide (57) comprend un matériau porteur poreux (60), et dans lequel le substrat liquide formant aérosol (58) est prévu sur le matériau porteur poreux (60).

6. Système de génération d'aérosol (10) selon l'une quelconque des revendications précédentes, dans lequel le dispositif de génération d'aérosol (12) comprend en outre un élément de transfert de liquide (62) configuré pour transférer le substrat liquide formant aérosol (58) vers le dispositif de chauffage électrique (64) pendant l'utilisation du système de génération d'aérosol (10).

7. Système de génération d'aérosol (10) selon la revendication 1, 2 ou 3, dans lequel la cartouche (18) comprend le substrat liquide formant aérosol (58) positionné dans le logement de cartouche (28).

8. Système de génération d'aérosol (200) selon la revendication 6, dans lequel la cartouche (218) comprend un matériau porteur poreux (260), et dans lequel le substrat liquide formant aérosol (58) est prévu sur le matériau porteur poreux (260).

9. Système de génération d'aérosol (200) selon la revendication 8, dans lequel le dispositif générateur d'aérosol (212) comprend en outre un élément de transfert de liquide (62) configuré pour transférer le substrat liquide formant aérosol (58) du matériau porteur poreux (260) vers le dispositif de chauffage électrique (64) pendant l'utilisation du système de génération d'aérosol (200) .

10. Système de génération d'aérosol (200) selon la revendication 9, dans lequel la cartouche (218) comprend en outre un joint cassable (248, 250), et dans lequel le dispositif générateur d'aérosol (212) comprend en outre un élément de perçage configuré pour percer le joint cassable (248, 250) lorsque le dispositif générateur d'aérosol (212) reçoit la cartouche (218).

11. Système de génération d'aérosol (200) selon la revendication 10, dans lequel l'élément de perçage comprend une partie d'arbre creuse (232) et une partie de perçage (234) à une extrémité de la partie d'arbre creuse (232), et dans lequel au moins une partie du dispositif de chauffage électrique (64) est positionné à l'intérieur de la partie d'arbre creuse (232).

12. Système de génération d'aérosol (200) selon la revendication 11, dans lequel une première partie de l'élément de transfert de liquide (62) est positionnée à l'intérieur de la partie d'arbre creuse (232), et dans lequel le dispositif de chauffage électrique (64) comprend une bobine de chauffage résistive enroulé au moins partiellement autour de la première partie de l'élément de transfert de liquide (62).

13. Système de génération d'aérosol (200) selon la revendication 12, dans lequel l'élément de transfert de liquide (62) s'étend à travers une ouverture dans la partie d'arbre creuse (232), et dans lequel une deuxième partie de l'élément de transfert de liquide (62) recouvre une surface extérieure de la partie d'arbre creuse (232) .

14. Système de génération d'aérosol (200) selon la revendication 13, dans lequel le matériau porteur poreux (260) a une forme annulaire définissant un passage (254) à travers le matériau porteur poreux (260), dans lequel le système de génération d'aérosol (200) est configuré de sorte que l'élément de perçage soit reçu au moins partiellement dans le passage (254) lorsque le dispositif générateur d'aérosol (212) reçoit la cartouche (218), et dans lequel le système de génération d'aérosol (200) est configuré de telle sorte que la deuxième partie de l'élément de transfert de liquide (62) entre en contact avec une surface intérieure du matériau porteur poreux (260) lorsque la partie de perçage (234) est reçue au moins partiellement à l'intérieur du passage (254).

15. Système de génération d'aérosol (200) selon la revendication 14, dans lequel la cartouche (218) comprend un canal d'écoulement d'air positionné entre le matériau porteur poreux (260) et le logement de cartouche (28), et dans lequel une extrémité en aval du canal d'écoulement d'air est en communication fluidique avec le substrat solide formant aérosol (38).
